# FASCICULE DE BREVET EUROPEEN

(11) **EP 3 072 870 B1**
(45) Date de publication et mention de la délivrance du brevet: **22.04.2020**
(21) Numéro de dépôt: 16165721.8
(22) Date de dépôt: 01.06.2010
(51) Int. Cl.: C07C 17/25, C07C 21/18

(54) **PROCEDE DE PREPARATION DE 2,3,3,3-TÉTRAFLUORO-1-PROPÈNE**
VERFAHREN ZUR HERSTELLUNG VON 2,3,3,3-TETRAFLUORO-1-PROPEN
METHOD FOR PREPARING 2,3,3,3-TETRAFLUORO-1-PROPENE

(30) Priorité: 23.07.2009 FR 0955139
(43) Date de publication de la demande: 28.09.2016
(62) Demande divisionnaire de: 10164600.8
(73) Titulaire: ARKEMA FRANCE, 92700 Colombes (FR)
(72) Inventeur: AVRIL, Karine, 69006 Lyon (FR); COLLIER, Bertrand, 69230 Saint-Genis-Laval (FR)
(74) Mandataire: Leca, François Michel

(56) Documents cités:
- EP-A1- 0 644 173
- WO-A1-2008/002500
- WO-A1-2009/084703
- WO-A1-2009/118632
- WO-A1-2009/138764
- WO-A1-2010/029240
- WO-A2-2008/030440
- WO-A2-2008/075017
- US-A1- 2007 179 324
- KNUNYANTS I L ET AL: "Reactions of fluoro olefins. XIII. Catalytic hydrogenation of perfluoro olefins", IZVESTIA AKADEMII NAUK SSSR. SERIA HIMICESKAA, MOSCOW, RU, 1 janvier 1960 (1960-01-01), pages 1312-1318, XP002548816, ISSN: 0002-3353
- Zyhowski et al.: "An Overview Of The Properties And Applicationsof HFC-245fa", Purdue University , 2002, Retrieved from the Internet: URL:https://docs.lib.purdue.edu/cgi/viewco ntent.cgi?referer=https://www.google.com/& httpsredir=1&article=1558&context=iracc [retrieved on 2018-07-18]
- Unknown author: "List of refrigerants", Wikipedia , Retrieved from the Internet: URL:https://en.wikipedia.org/wiki/List_of_ refrigerants [retrieved on 2018-07-18]

## Description

L'invention a pour objet un procédé de préparation de composés fluorés, à savoir le composé fluoré 2,3,3,3-tétrafluoro-1-propène.

Les hydrofluorocarbones (HFC) et en particulier les hydrofluorooléfines, telles que le 2,3,3,3-tétrafluoro-1-propène (HFO 1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. A la différence des CFC et des HCFC, qui sont potentiellement dangereux pour la couche d'ozone, les HFOs ne contiennent pas de chlore et donc ne posent pas de problème pour la couche d'ozone.

On connaît plusieurs procédés de fabrication du 1234yf.

WO2008/002499 décrit un procédé de production d'un mélange de 2,3,3,3-tétrafluoro-1-propène (HFO-1234yf) et de 1,3,3,3-tétrafluoro-1-propène (HFO-1234ze) par pyrolyse de 1,1,1,2,3-pentafluoropropane (HFC-245eb).

WO2008/002500 décrit un procédé de production d'un mélange de 2,3,3,3-tétrafluoro-1-propène (HFO-1234yf) et de 1,3,3,3-tétrafluoro-1-propène (HFO-1234ze) par conversion catalytique de 1,1,1,2,3-pentafluoropropane (HFC- 245eb) sur un catalyseur de déhydrofluoration.

Ces deux demandes précitées visent donc la production d'un mélange contenant une partie substantielle de produit HFO-1234ze.

WO2007/056194 décrit la préparation de HFO-1234yf par déhydrofluoration de HFC-245eb, soit avec de la potasse, typiquement une solution aqueuse d'au plus 50 % en poids de KOH, soit en phase gazeuse en présence d'un catalyseur, notamment catalyseur à base de nickel, de carbone ou une combinaison de ceux-ci.

Le document Knunyants et al, Journal of the USSR Academy of Sciences, Chemistry Department, "reactions of fluoro-olefins", report 13., "catalytic hydrogénation of perfluoro-olefins", 1960, décrit de façon distincte diverses réactions chimiques sur des composes fluorés. Ce document décrit l'hydrogénation sensiblement quantitative de HFP sur un catalyseur à base de palladium supporté sur alumine, la température passant de 20°C à 50°C, puis étant maintenue à cette valeur. Ce document décrit la déhydrofluoration du 1,1,1,2,3,3-hexafluoropropane (HFC-236ea) par passage au travers d'une suspension de KOH dans l'éther de dibutyle, pour produire du 1,2,3,3,3-pentafluoropropène-1 (HFO-1225ye) avec un rendement de 60 % seulement. Ce document décrit l'hydrogénation du 1,2,3,3,3-pentafluoropropène-1 (HFO-1225ye) en 1,1,1,2,3-pentafluoropropane (HFC-245eb) sur un catalyseur palladium supporté sur alumine. Au cours de cette hydrogénation il se produit aussi une réaction d'hydrogénolyse, une quantité significative de 1,1,1,2-tetrafluoropropane étant produite. Ce document décrit la déhydrofluoration de 1,1,1,2,3-pentafluoropropane (HFC-245eb) en 2,3,3,3-tétrafluoro-1-propène (HFO-1234yf) par passage dans une suspension de KOH en poudre dans l'éther de dibutyle avec un rendement de 70 % seulement. Ces réactions sont décrites indépendamment les unes des autres, mêmes s'il est indiqué qu'il est possible de les combiner pour synthétiser une gamme de dérivés d'éthylène, propylène et isobutylène contenant des quantités variables de fluor.

Le document US-P-5396000 décrit la préparation de 1,1,1,2,3-pentafluoropropane par déhydrofluoration catalytique de 1,1,1,2,3,3-hexafluoropropane (HFC-236ea) en 1,2,3,3,3-pentafluoropropène-1 (HFO-1225ye), suivi d'une hydrogénation pour produire le composé recherché. La déshydrohalogénation du HFC-236ea en HFO-1225ye est effectuée en phase gazeuse, le produit de la réaction étant, dans un exemple, envoyé directement au réacteur suivant dans lequel a lieu l'hydrogénation du composé HFO-1225ye en composé HFC-245eb. Il est aussi indiqué dans ce document que le composé HFC-236ea peut être obtenu par hydrogénation de hexafluoropropylène (HFP).

Le document US-P-5679875 décrit la préparation de 1,1,1,2,3-pentafluoropropane par déhydrofluoration catalytique de 1,1,1,2,3,3-hexafluoropropane (HFC-236ea) en 1,2,3,3,3-pentafluoropropène-1 (HFO-1225ye), suivi d'une hydrogénation pour produire le composé recherché. Les réactions sont effectuées en phase gaz. Il est aussi indiqué dans ce document que le composé HFC-236ea peut être obtenu par hydrogénation de hexafluoropropylène (HFP).

Le document WO 2008/030440 décrit la préparation du HFO-1234yf à partir du HFO-1225ye en faisant réagir le HFO-1225ye avec de l'hydrogène en présence d'un catalyseur pour donner le HFC-245eb, puis en faisant réagir le HFC-245eb avec une solution aqueuse basique en présence d'un catalyseur de transfert de phase et un solvant non-aqueux, non-alcoolique.

Le document WO 2008/075017 illustre la réaction de déhydrofluoration du 1,1,1,2,3,3 hexafluoropropane (HFC-236ea) en 1,1,1,2,3 pentafluoropropène (HFO-1225ye) à 150°C en présence d'une solution aqueuse de 50 % en poids de KOH. En l'absence d'un catalyseur de transfert de phase, la conversion au bout de 3 heures et demie est de 57,8 % et la sélectivité en HFO-1225ye est de 52,4 % (essai 1). En présence d'un catalyseur de transfert de phase, cette conversion est atteinte au bout de 2,5 heures seulement et la sélectivité est pratiquement inchangée (essai 4). Comme indiqué au tableau 2 de ce document pour accroître la sélectivité en HFO-1225ye, il est nécessaire d'utiliser un solvant organique.

Il existe un besoin d'un procédé de préparation de HFO-1234yf de haute pureté à partir d'un produit de départ qui soit aisément accessible, et qui conduise au produit recherché avec une sélectivité élevée, de préférence un rendement élevé et avantageusement une productivité élevée.

La demanderesse a remarqué que certains sous-produits générés dans les différentes étapes d'un procédé de fabrication du HFO-1234yf, notamment ceux ayant un point d'ébullition voisin de celui du HFO-1234yf, se séparent difficilement du HFO-1234yf et nécessitent des conditions très sévères qui sont généralement coûteuses.

De même, la séparation de certains de réactifs non réagis dans les différentes étapes ou des produits intermédiaires dans le procédé sont difficilement séparables du HFO-1234yf. On peut citer à titre d'exemple l'HFP.

La présente invention fournit donc un procédé de fabrication du HFO-1234yf de haute pureté ne présentant pas les inconvénients précités.

Le procédé de préparation de 2,3,3,3-tétrafluoro-1-propène, selon la présente invention, comprend les étapes suivantes:
(i) hydrogénation en phase gazeuse de hexafluoropropylène en 1,1,1,2,3,3-hexafluoropropane en présence d'une quantité surstoechiométrique d'hydrogène et d'un catalyseur dans un réacteur;
(ii) déhydrofluoration du 1,1,1,2,3,3-hexafluoropropane obtenu à l'étape précédente en 1,2,3,3,3-pentafluoropropène-1 en présence d'un catalyseur de déhydrofluoration ou à l'aide d'un mélange eau et hydroxyde de potassium;
(iii) hydrogénation en phase gazeuse du 1,2,3,3,3-pentafluoropropène-1 obtenu à l'étape précédente en 1,1,1,2,3-pentafluoropropane en présence d'une quantité surstœchiométrique d'hydrogène et d'un catalyseur dans un réacteur;
(iv) purification du 1,1,1,2,3-pentafluoropropane obtenu à l'étape précédente pour obtenir un flux comprenant plus de 98% en poids de 1,1,1,2,3-pentafluoropropane,
(v) déhydrofluoration du 1,1,1,2,3-pentafluoropropane purifié en 2,3,3,3-tétrafluoro-1-propène en présence d'un catalyseur de déhydrofluoration ou à l'aide d'un mélange eau et hydroxyde de potassium;
(vi) purification du 2,3,3,3-tétrafluoro-1-propène obtenu à l'étape précédente.

Le procédé, selon la présente invention, peut comprendre en outre au moins l'une des caractéristiques suivantes:
- le(s) étape(s) d'hydrogénation (i) et/ou (iii) est (sont) mise(s) en œuvre dans un réacteur adiabatique mono ou multiétagé ou dans au moins deux réacteurs adiabatiques en série ;
- une partie de l'effluent gazeux issu de l'étape d'hydrogénation (i) comprenant du HFC-236ea et de l'hydrogène non réagi est recyclé à l'étape (i) ;
- l'effluent gazeux issu de l'étape d'hydrogénation (i) est soumis à une étape de condensation dans les conditions telles que l'hydrogène non réagi n'est pas condensé et qu'une partie du HFC-236ea formé à l'étape (i) est condensé ;
- une partie de l'effluent gazeux issu de l'étape d'hydrogénation (iii) comprenant du HFC-245eb et de l'hydrogène non réagi est recyclé à l'étape (iii) ;
- l'effluent gazeux issu de l'étape d'hydrogénation (iii) est soumis à une étape de condensation dans les conditions telles que l'hydrogène non réagi n'est pas condensé et qu'une partie du HFC-245eb formé à l'étape (i) est condensé ;
- l'hydroxyde de potassium est présent dans le milieu réactionnel de l'étape (ii) et/ou (v) en quantité comprise entre 20 et 75 % en poids et avantageusement comprise entre 55 et 70 % en poids par rapport au poids du mélange eau et KOH ;
- une étape de traitement du fluorure de potassium, co-produit dans l'étape de déhydrofluoration (ii) et/ou (v) avec de l'hydroxyde de calcium ;
- une étape de purification du 1,2,3,3,3-pentafluoropropène-1 obtenu à l'étape de déhydrofluoration (ii) ;
- l'étape (vi) forme un flux comprenant plus de 99,8% en poids de 2,3,3,3-tetrafluoropropene ;
- les composés à éliminer sont choisis parmi le 1,2,3,3,3-pentafluoropropène, le 1,1,3,3,3-pentafluoropropène et le 1,1,12-tetrafluoroethane.

Est divulguée également une composition comprenant plus de 99,5% en poids de 2,3,3,3-tétrafluoropropène, de préférence plus de 99,8 % en poids. La composition peut être obtenue par le procédé selon la présente invention. La composition peut comprendre moins de 0,5% en poids d'impuretés légères et d'impuretés lourdes. Les impuretés légères peuvent comprendre le trifluoropropyne. Les impuretés lourdes peuvent comprendre le 1,1,1,2,3-pentafluoropropane.

La pureté du 2,3,3,3-tétrafluoro-1-propène obtenue selon le procédé de la présente invention est de préférence supérieure à 99,5% en poids et avantageusement supérieure à 99,8 % en poids.

L'étape de purification (iv) a pour but de purifier le flux comprenant du HFC-245eb provenant de l'étape d'hydrogénation (iii) en vue d'obtenir du HFC-245eb, de pureté de préférence supérieure à 98% en poids, destiné à l'étape de déhydrofluoration (v).

Cette étape de purification consiste, de préférence, à éliminer des composés qui ont une température d'ébullition proche (plus ou moins de 10°C) de celle du HFO-1234yf ou à éliminer des précurseurs desdits composés, ayant une température d'ébullition d'au moins 20°C inférieure, voire d'au moins 10°C inférieure par rapport à celle du HFC-245eb.

Les composés à éliminer à l'étape de purification (iv) sont, de préférence, choisis parmi l'hexafluoropropène, le cyclohexafluoropropène, le 1,2,3,3,3-pentafluoropropène (isomères Z et E), le 1,1,3,3,3-pentafluoropropène (HFO-1225zc), le 1,1,1,2-tetrafluoropropane (HFC-254eb), le 3,3,3-trifluoropropène (HFO-1243zf), le 1,1,1,2-tetrafluoroethane(HFC-134a), le 1,1,2-trifluoroethane (HFC-143) et le 1,1,1,2,3,3-hexafluoropropane.

Les composés à éliminer à l'étape de purification (iv) sont, avantageusement, choisis parmi le 1,2,3,3,3-pentafluoropropène (isomères Z et E), le 1,1,3,3,3-pentafluoropropène (HFO-1225zc) et le 1,1,1,2-tetrafluoroethane(HFC-134a).

Le procédé, selon la présente invention, peut être mis en œuvre en discontinu, semi-continu ou continu. De préférence, le procédé selon la présente invention est mis en œuvre en continu et le flux provenant de l'étape précédente étant directement envoyé à l'étape suivante, après éventuelle purification. On obtient ainsi un procédé économique de préparation du composé HFO-1234yf de haute pureté, le produit de départ, HFP, étant disponible facilement sur le marché, à un coût faible.

### Etape d'hydrogénation (i)

L'étape d'hydrogénation peut être mise en œuvre en présence d'un rapport molaire H_{2/}HFP compris entre 1,1 et 40, de préférence comprise entre 2 et 15.

L'étape d'hydrogénation peut être mise en œuvre à une pression comprise entre 0,5 et 20 bars absolu et de préférence entre 1 et 5 bars absolu.

Comme catalyseurs susceptibles d'être utilisés dans l'étape d'hydrogénation, on peut notamment citer les catalyseurs à base d'un métal du groupe VIII ou rhénium, éventuellement supporté, par exemple sur carbone, carbure de silicium, alumine, fluorure d'aluminium.

Comme métal, on peut utiliser du platine ou du palladium, en particulier du palladium, avantageusement supporté sur carbone ou alumine. On peut aussi associer ce métal avec un autre métal comme l'argent, le cuivre, l'or, le tellure, le zinc, le chrome, le molybdène et le thallium.

Le catalyseur peut être présent sous toute forme appropriée, extrudés, pastilles ou billes.

De préférence, on utilise un catalyseur comprenant entre 0,05 et 10% en poids et avantageusement entre 0,1 et 5% en poids de palladium supporté sur de l'alumine ou carbone.

L'étape d'hydrogénation peut être mise en œuvre dans des conditions telles que la température à l'entrée du réacteur est comprise entre 30 et 200 °C, de préférence entre 30 et 100°C et celle à la sortie du réacteur est comprise entre 50 et 250°C, de préférence entre 80 et 150 °C.

Le temps de contact (rapport entre le volume de catalyseur et le flux total gazeux dans les conditions normales de température et de pression) est de préférence entre compris entre 0,2 et 10 secondes et avantageusement entre 1 et 5 secondes.

Cette étape d'hydrogénation peut être mise en œuvre dans un réacteur adiabatique, éventuellement multiétagé ou dans au moins deux réacteurs adiabatiques en série.

L'étape d'hydrogénation de l'HFP est sensiblement quantitative.

De préférence, une partie de l'effluent gazeux issu de l'étape d'hydrogénation (i) comprenant du HFC-236ea et de l'hydrogène non réagi est recyclé à l'étape (i).

Avantageusement, l'effluent gazeux issu de l'étape d'hydrogénation (i) est soumis à une étape de condensation dans des conditions telles que l'hydrogène non réagi n'est pas condensé et qu'une partie du HFC-236ea formé à l'étape (i) est condensé.

De préférence, l'étape de condensation est mise en œuvre à une température comprise entre 0 et 50°C et à une pression comprise entre 0,5 et 20 bar absolu, avantageusement entre 1 et 5 bars absolu.

De préférence, l'étape de condensation est mise en œuvre dans des conditions telles qu'entre 1 et 30 % du HFC-236ea en sortie de l'étape (i) est condensé et avantageusement entre 2 et 10 % est condensé.

La fraction non condensée est ensuite recyclée à l'étape d'hydrogénation (i) après un éventuel chauffage.

### Etape de déhydrofluoration (ii)

Le HFC-236ea formé à l'étape (i), après éventuelle condensation et vaporisation, est ensuite soumise à une étape de déhydrofluoration, de préférence à l'aide de l'hydroxyde de potassium et avantageusement mise en œuvre dans un réacteur agité. L'hydroxyde de potassium est, de préférence présent dans le milieu réactionnel en quantité comprise entre 20 et 75 % en poids et avantageusement comprise entre 55 et 70 % en poids par rapport au poids du mélange eau et KOH.

Le milieu réactionnel aqueux de l'étape de déhydrofluoration, comprenant du KOH, est de préférence maintenu à une température comprise entre 80 et 180 °C, avantageusement comprise entre 125 et 180°C. Une température du milieu réactionnel particulièrement préférée est comprise entre 145 et 165°C.

L'étape de déhydrofluoration à l'aide du KOH peut être mise en œuvre à une pression de 0,5 à 20 bars mais on préfère travailler à une pression comprise entre 0,5 et 5 bars absolus et plus avantageusement entre 1,1 et 2,5 bars absolus.

Au cours de l'étape de déhydrofluoration à l'aide de KOH, il se forme du fluorure de potassium.

Le procédé selon la présente invention peut comprendre une étape de traitement au cours de laquelle le fluorure de potassium co-produit à l'étape de déhydrofluoration est mis en contact avec de l'hydroxyde de calcium dans un milieu réactionnel aqueux à une température de préférence comprise entre 50 et 150 °C et avantageusement comprise entre 70 et 120°C et plus avantageusement entre 70 et 100°C.

Cette étape de traitement est de préférence mise en œuvre en introduisant de l'hydroxyde de calcium dans un réacteur contenant une partie du milieu réactionnel, provenant de l'étape de déhydrofluoration, comprenant du fluorure de potassium, de l'hydroxyde de potassium et de l'eau, après éventuelle dilution.

Le fluorure de potassium est de préférence présent entre 4 et 45 % en poids et avantageusement de 7 et 20 % en poids par rapport au milieu réactionnel provenant de l'étape de déhydrofluoration.

Le milieu réactionnel du traitement comprend de préférence entre 4 et 50% en poids d'hydroxyde de potassium et avantageusement entre 10 et 35 % en poids d'hydroxyde de potassium par rapport au poids total d'hydroxyde de potassium et eau dans le milieu.

L'étape de traitement avec l'hydroxyde de calcium permet de régénérer de l'hydroxyde de potassium qui peut être recyclé à l'étape de déhydrofluoration et d'obtenir du fluorure de calcium de qualité commerciale valorisable après séparation par exemple filtration et décantation.

Du fluorure de calcium de taille moyenne comprise entre 20 et 35 µm (taille moyenne à 50 % en poids de la distribution granulométrique)est obtenu dans les conditions préférées de cette étape de traitement.

L'étape de traitement à l'hydroxyde de calcium peut être mise en œuvre dans tout type de réacteur connu de l'homme de l'art, par exemple un réacteur agité.

### Etape d'hydrogénation (iii)

Le HFO-1225ye obtenu à l'étape (ii), éventuellement après purification, est soumise à une étape d'hydrogénation en présence d'un catalyseur.

La purification du HFO-1225ye peut comprendre une double distillation avec élimination des impuretés légères dans la première et élimination des impuretés lourdes dans la deuxième distillation.

L'étape d'hydrogénation peut être mise en œuvre en présence d'un rapport molaire H₂/HFO-1225ye compris entre 1,1 et 40, de préférence comprise entre 2 et 15.

L'étape d'hydrogénation peut être mise en œuvre à une pression comprise entre 0,5 et 20 bars absolu et de préférence entre 1 et 5 bars absolu.

Comme catalyseurs susceptibles d'être utilisés dans l'étape d'hydrogénation, on peut notamment citer les catalyseurs à base d'un métal du groupe VIII ou rhénium, éventuellement supporté, par exemple sur carbone, carbure de silicium, alumine, fluorure d'aluminium.

Comme métal, on peut utiliser du platine ou du palladium, en particulier du palladium, avantageusement supporté sur carbone ou alumine. On peut aussi associer ce métal avec un autre métal comme l'argent, le cuivre, l'or, le tellure, le zinc, le chrome, le molybdène et le thallium.

Le catalyseur peut être présent sous toute forme appropriée, extrudés, pastilles ou billes.

De préférence, on utilise un catalyseur comprenant entre 0,05 et 10% en poids et avantageusement entre 0,1 et 5% en poids de palladium supporté sur de l'alumine ou carbone.

L'étape d'hydrogénation peut être mise en œuvre dans des conditions telles que la température à l'entrée du réacteur est comprise entre 50 et 200 °C, de préférence entre 80 et 140°C et celle à la sortie du réacteur est comprise entre 80 et 250°C, de préférence entre 110 et 160 °C.

Le temps de contact (rapport entre le volume de catalyseur et le flux total gazeux dans les conditions normales de température et de pression) est de préférence entre compris entre 0,2 et 10 secondes et avantageusement entre 1 et 5 secondes.

Cette étape d'hydrogénation peut être mise en œuvre dans un réacteur adiabatique, éventuellement multiétagé ou dans au moins deux réacteurs adiabatiques en série.

L'étape d'hydrogénation du HFO-1225ye est sensiblement quantitative.

De préférence, une partie de l'effluent gazeux issu de l'étape d'hydrogénation (iii) comprenant du HFC-245eb et de l'hydrogène non réagi est recyclé à l'étape (iii).

Avantageusement, l'effluent gazeux issu de l'étape d'hydrogénation (iii) est soumis à une étape de condensation dans des conditions telles que l'hydrogène non réagi n'est pas condensé et qu'une partie du HFC-245eb formé à l'étape (iii) est condensé.

De préférence, l'étape de condensation est mise en œuvre à une température comprise entre 0 et 50°C et à une pression comprise entre 0,5 et 20 bar absolu, avantageusement entre 1 et 5 bars absolu.

De préférence, l'étape de condensation est mise en œuvre dans des conditions telles qu' entre 1 et 30 % du HFC-245eb en sortie de l'étape (iii) est condensé et avantageusement entre 2 et 10 % est condensé.

La fraction non condensée est ensuite recyclée à l'étape d'hydrogénation (iii) après un éventuel chauffage.

### Etape de purification (iv)

Le HFC-245eb obtenu à l'étape d'hydrogénation (iii), après éventuelle condensation, est soumis à une étape de purification en vue d'obtenir du HFC-245eb, de pureté de préférence supérieure à 98% en poids, destiné à l'étape de déhydrofluoration (v).

Cette étape de purification consiste de préférence à éliminer des composés qui ont une température d'ébullition proche de celle du HFO-1234yf ou des précurseurs desdits composés, ayant une température d'ébullition d'au moins 20°C inférieure, voire d'au moins 10°C inférieure par rapport à celle du HFC-245eb.

Ces composés pouvant être des sous-produits des différentes étapes du procédé, des réactifs non réagis des différentes étapes et/ou des intermédiaires du procédé.

Cette étape de purification comprend de préférence au moins une étape de distillation classique mise en œuvre à une pression comprise entre 1 et 20 bar absolu et avantageusement entre 5 et 10 bar absolu.

### Etape de déhydrofluoration (v)

Le HFC-245eb purifié à l'étape (iv), de pureté de préférence supérieure à 98% en poids, est ensuite soumise à une étape de déhydrofluoration, de préférence à l'aide de l'hydroxyde de potassium et avantageusement mise en œuvre dans un réacteur agité. L'hydroxyde de potassium est, de préférence présent dans le milieu réactionnel en quantité, comprise entre 20 et 75 % en poids et avantageusement comprise entre 55 et 70 % en poids par rapport au poids du mélange eau et KOH.

Le milieu réactionnel aqueux, comprenant du KOH, de l'étape de déhydrofluoration est de préférence maintenu à une température comprise entre 80 et 180 °C, avantageusement comprise entre 125 et 180°C. Une température du milieu réactionnel particulièrement préférée est comprise entre 145 et 165°C.

L'étape de déhydrofluoration peut être mise en œuvre à une pression de 0,5 à 20 bars mais on préfère travailler à une pression comprise entre 0,5 et 5 bars absolus et plus avantageusement entre 1,1 et 2,5 bars absolus.

Au cours de l'étape de déhydrofluoration à l'aide de KOH, il se forme du fluorure de potassium.

Le procédé selon la présente invention peut comprendre une étape de traitement au cours de laquelle le fluorure de potassium co-produit à l'étape de déhydrofluoration est mis en contact avec de l'hydroxyde de calcium dans un milieu réactionnel aqueux à une température de préférence comprise entre 50 et 150 °C et avantageusement comprise entre 70 et 120°C et plus avantageusement entre 70 et 100°C.

Cette étape de traitement est de préférence mise en œuvre en introduisant de l'hydroxyde de calcium dans un réacteur contenant une partie du milieu réactionnel, provenant de l'étape de déhydrofluoration, comprenant du fluorure de potassium, de l'hydroxyde de potassium et de l'eau après éventuelle dilution.

Le fluorure de potassium est de préférence présent entre 4 et 45 % en poids et avantageusement de 7 et 20 % en poids par rapport au milieu réactionnel provenant de l'étape de déhydrofluoration.

Le milieu réactionnel du traitement comprend de préférence entre 4 et 50% en poids d'hydroxyde de potassium et avantageusement entre 10 et 35 % en poids d'hydroxyde de potassium par rapport au poids total d'hydroxyde de potassium et eau dans le milieu.

L'étape de traitement avec l'hydroxyde de calcium permet de régénérer de l'hydroxyde de potassium qui peut être recyclé à l'étape de déhydrofluoration et d'obtenir du fluorure de calcium de qualité commerciale valorisable après séparation par exemple filtration et décantation.

Du fluorure de calcium de taille moyenne comprise entre 20 et 35 µm (taille moyenne à 50 % en poids de la distribution granulométrique)est obtenu dans les conditions préférées de cette étape de traitement.

L'étape de traitement à l'hydroxyde de calcium peut être mise en œuvre dans tout type de réacteur connu de l'homme de l'art, par exemple un réacteur agité.

### Etape de purification (vi)

Le flux gazeux à l'issue de l'étape de déhydrofluoration (v) comprenant le HFO-1234yf, le HFC-245eb non réagi et les sous produits est soumis à une étape de purification pour obtenir de l'HFO-1234yf ayant une pureté d'au moins de 99,5% en poids, de préférence d'au moins 99,8% en poids.

La purification comprend de préférence en une première étape de distillation pour séparer les impuretés légères, notamment le trifluoropropyne et une deuxième étape distillation pour séparer le HFO-1234yf des impuretés lourdes, notamment le HFC-245eb.

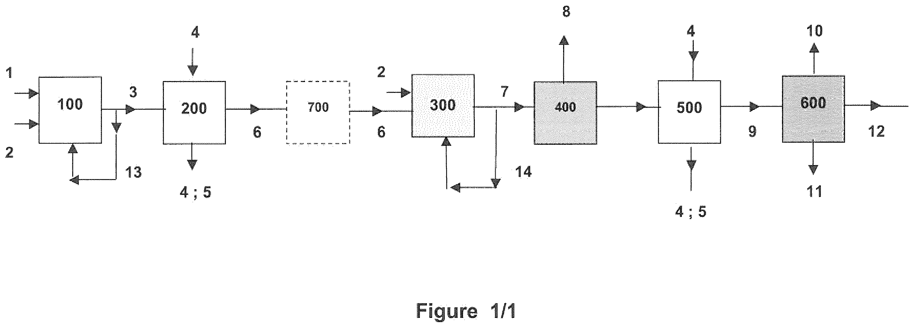

Selon un mode de réalisation (Figure 1) particulièrement préféré de l'invention, (i) on fait réagir en continu de l'hexafluoropropylène (1) avec de l'hydrogène (2) en phase gazeuse en quantité surstœchiométrique, dans un réacteur adiabatique (100), en présence d'un catalyseur pour donner du 1,1,1,2,3,3-hexafluoropropane et on recycle une partie (13) de l'effluent gazeux issu de cette réaction; (ii) on fait réagir la partie non recyclée (3) de l'effluent issu de l'étape (i), comprenant du 1,1,1,2,3,3-hexafluoropropane, avec de l'hydroxyde de potassium (4) présent dans un milieu réactionnel aqueux (200), en quantité comprise entre 20 et 75 % en poids et de préférence comprise entre 55 et 70 % en poids par rapport au poids du mélange eau et KOH du milieu réactionnel, maintenu à une température comprise entre 80 et 180 °C, de préférence comprise entre 145 et 165°C pour donner du 1,2,3,3,3-pentafluoropropène-1 et du fluorure de potassium ; (iii) on fait réagir le 1,2,3,3,3-pentafluoropropène-1 (6) obtenu à l'étape (ii) avec de l'hydrogène (2) en phase gazeuse en quantité surstœchiométrique, dans un réacteur adiabatique (300), en présence d'un catalyseur pour donner du 1,1,1,2,3-pentafluoropropane et on recycle une partie (14) de l'effluent gazeux issu de cette réaction ; (iv) on soumet la partie non recyclée (7) de l'effluent issu de l'étape (iii), comprenant du 1,1,1,2,3-pentafluoropropane, à une étape de purification (400) pour éliminer des impuretés (8) et obtenir ainsi du 1,1,1,2,3-pentafluoropropane, de pureté de préférence supérieure à 98% en poids; (v) on fait réagir du 1,1,1,2,3-pentafluoropropane obtenu après purification de l'étape (iv) avec de l'hydroxyde de potassium (4) présent dans un milieu réactionnel aqueux (500), en quantité comprise entre 20 et 75 % en poids et de préférence comprise entre 55 et 70 % en poids par rapport au poids du mélange eau et KOH du milieu réactionnel, maintenu à une température comprise entre 80 et 180 °C, de préférence comprise entre 145 et 165°C pour donner du 2,3,3,3-pentafluoropropène-1 et du fluorure de potassium et (vi) le flux gazeux (9) à l'issue de l'étape de déhydrofluoration (v) comprenant le HFO-1234yf, le HFC-245eb non réagi et les sous produits est soumis à une étape de purification (600) pour obtenir de l'HFO-1234yf (12) ayant une pureté supérieure à 99,5 % en poids, de préférence supérieure à 99,8 % en poids. Les sous-produits légers (10) séparés du HFO-1234yf peuvent être soumis à un traitement et le HFC-245eb non réagi (11) peut être recyclé en (500).

La purification de l'étape (iv) comprend de préférence une distillation classique à une pression comprise entre 1 et 20 bar absolu.

Le fluorure de potassium (5) formé à l'étape (ii) et (v) peut être soumis à un traitement par de l'hydroxyde de calcium, dans les conditions décrites ci-dessus, pour régénérer de l'hydroxyde de potassium et former du fluorure de calcium.

Le HFO-1225ye obtenu à l'étape (ii) peut être soumis à une étape de purification (700), de préférence à une double distillation, avant la réaction d'hydrogénation de l'étape (iii).

L'effluent gazeux à l'issue de l'étape d'hydrogénation (i) et/ou (iii) peut être partiellement condensé, la partie non condensée comprenant la totalité de l'hydrogène non réagi est recyclée.

Les étapes d'hydrogènation (i) et (iii) peuvent être mises en œuvre dans les conditions décrites ci-dessus.

Selon un mode de réalisation 1, le procédé de préparation de 2,3,3,3-tétrafluoro-1-propène comprenant les étapes suivantes:
(i) hydrogénation en phase gazeuse de hexafluoropropylène en 1,1,1,2,3,3-hexafluoropropane en présence d'une quantité surstoechiométrique d'hydrogène et d'un catalyseur dans un réacteur;
(ii) déhydrofluoration du 1,1,1,2,3,3-hexafluoropropane obtenu à l'étape précédente en 1,2,3,3,3-pentafluoropropène-1 en présence d'un catalyseur de déhydrofluoration ou à l'aide d'un mélange eau et hydroxyde de potassium;
(iii) hydrogénation en phase gazeuse du 1,2,3,3,3-pentafluoropropène-1 obtenu à l'étape précédente en 1,1,1,2,3-pentafluoropropane en présence d'une quantité surstoechiométrique d'hydrogène et d'un catalyseur dans un réacteur;
(iv) purification du 1,1,1,2,3-pentafluoropropane obtenu à l'étape précédente
(v) déhydrofluoration du 1,1,1,2,3-pentafluoropropane purifié en 2,3,3,3-tétrafluoro-1-propène en présence d'un catalyseur de déhydrofluoration ou à l'aide d'un mélange eau et hydroxyde de potassium;
(vi) purification du 2,3,3,3-tétrafluoro-1-propène obtenu à l'étape précédente.

Mode de réalisation 2 dans lequel le procédé selon le mode de réalisation 1 est caractérisé en la purification du 1,1,1,2,3-pentafluoropropane en (iv) comprend une étape de distillation à une pression comprise entre 1 et 20 bar absolu.

Mode de réalisation 3 dans lequel le procédé selon le mode de réalisation 1 ou 2 est caractérisé en ce que les composés à éliminer à l'étape (iv) ont une température d'ébullition d'au moins 20°C inférieure, voire d'au moins 10°C inférieure par rapport à celle du 1,1,1,2,3-pentafluoropropane.

Mode de réalisation 4 dans lequel le procédé selon l'un quelconque des modes de réalisation 1 à 3 est caractérisé en ce que l'hydroxyde de potassium représente entre 55 et 75 % en poids du mélange eau-hydroxyde de potassium de l'étape (ii) et/ou (v).

Mode de réalisation 5 dans lequel le procédé selon l'un quelconque des modes de réalisation 1 à 4 dans lequel l'étape (ii) et/ou l'étape (v) est ou sont mise(s) en œuvre à une température comprise entre 80 et 180°C, de préférence comprise entre 145 et 165°C.

Mode de réalisation 6 dans lequel le procédé selon l'un quelconque des modes de réalisation 1 à 5 est caractérisé en ce que le flux issu de l'étape (ii) est soumis à une étape de purification avant d'être envoyé à l'étape (iii).

Mode de réalisation 7 dans lequel le procédé selon le mode de réalisation 6 est caractérisé en ce que la purification comprend une double distillation.

Mode de réalisation 8 dans lequel le procédé selon l'un quelconque des modes de réalisation 1 à 7 est caractérisé en ce qu'il est mis en œuvre en continu.

Mode de réalisation 9 dans lequel le procédé selon l'un quelconque des modes de réalisation 1 à 8 est caractérisé en ce que l'étape (i) et/ou l'étape (iii) est ou sont mis en œuvre dans un réacteur adiabatique, éventuellement multiétagé ou dans au moins deux réacteurs adiabatiques en série.

Mode de réalisation 10 dans lequel le procédé selon l'un quelconque des modes de réalisation 1 à 9 est caractérisé en ce qu'une partie de l'effluent gazeux issu de l'étape (i) et/ou (iii) est recyclé.

Mode de réalisation 11 dans lequel le procédé selon l'un quelconque des modes de réalisation 1 à 10 est caractérisé en ce que l'effluent gazeux issu de l'étape (i) et/ou (iii) est ou sont soumis à une étape de condensation partielle.

Mode de réalisation 12 dans lequel le procédé selon l'un quelconque des modes de réalisation 1 à 11 est caractérisé en ce qu'il comprend une étape de traitement du fluorure de potassium, co-produit dans l'étape de déhydrofluoration (ii) et (v), avec de l'hydroxyde de calcium.

Mode de réalisation 13 dans lequel le procédé selon l'un quelconque des modes de réalisation 1 à 12 est caractérisé en ce que le catalyseur de l'étape (i) et/ou l'étape (iii) comprend entre 0,05 et 10 % en poids et de préférence, entre 0,1 et 5 % en poids de palladium supporté sur de l'alumine ou carbone.

Mode de réalisation 14 dans lequel le procédé selon l'un quelconque des modes de réalisation 1 à 13 est caractérisé en ce que l'étape (i) est mise en œuvre à une température à l'entrée du réacteur comprise entre 30 et 100°C.

Mode de réalisation 15 dans lequel le procédé selon l'un quelconque des modes de réalisation 1 à 14 est caractérisé en ce que l'étape (iii) est mise en œuvre à une température à l'entrée du réacteur comprise entre 80 et 140°C.

Mode de réalisation 16, 2,3,3,3-tétrafluoropropène de pureté supérieure à 99,5 % en poids, de préférence supérieure à 99,8 % en poids.

### PARTIE EXPERIMENTALE

On distille dans une colonne, à 40 plateaux théoriques, un mélange comprenant 1g d'hexafluoropropène, 1g du cyclohexafluoropropène, 1g du HFO-1234yf, 0,2g du HFC-134a, 1g du HFO-1243zf, 0,1g du HFO-1225zc, 10g du HFO-1225ye (Z ou E), 40g du HFC-254eb, 0,01g du HFC-143, 1g du HFC-236ea 0,5g d'acide hydrofluorhydrique et 944,19 g du HFC-245eb.

La distillation est mise en œuvre à 6 bar absolu, avec une température en pied de colonne d'environ 80°C et une température en tête de 50°C.

A la fin de la distillation, on analyse le fond de colonne et on trouve 0,001g du HFC-143, 0,094g du HFC-236ea et 938g du HFC-245eb. Les autres composés ont été éliminés en tête de colonne.

Le HFC-245eb distillé est ensuite soumis à une étape de déhydrofluoration dans un réacteur agité en présence d'un mélange eau et hydroxyde de potassium. Le mélange est maintenu à 150°C et le KOH dans le mélange représente 65% en poids par rapport au poids total eau/KOH.

Le HFO-1234yf formé est condensé puis soumis à une distillation dans une colonne maintenue dans un premier temps à une pression de 13 bar et une température en pied de 60°C, pour séparer le trifluoropropyne et ensuite à une pression de 11 bar et une température en tête de 50°C pour obtenir en tête du HFO-1234yf avec une pureté supérieure à 99,9% en poids.

## Revendications

1. Procédé de préparation de 2,3,3,3-tétrafluoro-1-propène comprenant les étapes suivantes:
(i) hydrogénation en phase gazeuse de hexafluoropropylène en 1,1,1,2,3,3-hexafluoropropane en présence d'une quantité surstœchiométrique d'hydrogène et d'un catalyseur dans un réacteur;
(ii) déhydrofluoration du 1,1,1,2,3,3-hexafluoropropane obtenu à l'étape précédente en 1, 2, 3, 3, 3-pentafluoropropène-1 en présence d'un catalyseur de déhydrofluoration ou à l'aide d'un mélange eau et hydroxyde de potassium;
(iii) hydrogénation en phase gazeuse du 1,2,3,3,3-pentafluoropropène-1 obtenu à l'étape précédente en 1,1,1,2,3-pentafluoropropane en présence d'une quantité surstoechiométrique d'hydrogène et d'un catalyseur dans un réacteur;
(iv) purification du 1,1,1,2,3-pentafluoropropane obtenu à l'étape précédente pour obtenir un flux comprenant plus de 98% en poids de 1,1,1,2,3-pentafluoropropane,
(v) déhydrofluoration du 1,1,1,2,3-pentafluoropropane purifié en 2,3,3,3-tétrafluoro-1-propène en présence d'un catalyseur de déhydrofluoration ou à l'aide d'un mélange eau et hydroxyde de potassium;
(vi) purification du 2,3,3,3-tétrafluoro-1-propène obtenu à l'étape précédente.

2. Procédé selon la revendication 1, **caractérisé en ce que** la purification du 1,1,1,2,3-pentafluoropropane en (iv) comprend une étape de distillation à une pression comprise entre 1 et 20 bar absolu.

3. Procédé selon la revendication 1 ou 2 **caractérisé en ce que** les composés à éliminer à l'étape (iv) ont une température d'ébullition d'au moins 20°C inférieure, voire d'au moins 10°C inférieure par rapport à celle du 1,1,1,2,3-pentafluoropropane.

4. Procédé selon la revendication précédente **caractérisé en ce que** les composés à éliminer sont choisis parmi le 1,2,3,3,3-pentafluoropropène, le 1,1,3,3,3-pentafluoropropène et le 1,1,12-tetrafluoroethane.

5. Procédé selon l'une quelconque des revendications précédentes **caractérisé en ce que** l'hydroxyde de potassium représente entre 20 et 75 % en poids du mélange eau-hydroxyde de potassium de l'étape (ii) et/ou (v).

6. Procédé selon l'une quelconque des revendications précédentes, dans lequel l'étape (ii) et/ou l'étape (v) est ou sont mise(s) en œuvre à une température comprise entre 80 et 180°C, de préférence comprise entre 145 et 165°C.

7. Procédé selon l'une quelconque des revendications précédentes **caractérisé en ce que** l'effluent gazeux issu de l'étape (i) et/ou (iii) est ou sont soumis à une étape de condensation partielle.

8. Procédé selon l'une quelconque des revendications précédentes **caractérisé en ce que** le catalyseur de l'étape (i) et/ou l'étape (iii) comprend entre 0,05 et 10 % en poids et de préférence, entre 0,1 et 5 % en poids de palladium supporté sur de l'alumine ou carbone.

9. Procédé selon l'une quelconque des revendications précédentes **caractérisé en ce que** l'étape (iii) est mise en œuvre à une température à l'entrée du réacteur comprise entre 80 et 140°C.

10. Procédé selon l'une quelconque des revendications précédentes **caractérisé en ce que** l'étape (vi) forme un flux comprenant plus de 99,8% en poids de 2,3,3,3-tetrafluoropropene.

## Patentansprüche

1. Verfahren zur Herstellung von 2,3,3,3-Tetrafluor-1-propen, das folgende Schritte umfasst:
(i) Gasphasenhydrierung von Hexafluorpropylen zu 1,1,1,2,3,3-Hexafluorpropan in Gegenwart einer überstöchiometrischen Menge Wasserstoff und eines Katalysators in einem Reaktor;
(ii) Dehydrofluorierung des im vorhergehenden Schritt erhaltenen 1,1,1,2,3,3-Hexafluorpropans zu 1,2,3,3,3-Pentafluor-1-propen in Gegenwart eines Dehydrofluorierungskatalysators oder mit Hilfe einer Mischung von Wasser und Kaliumhydroxid;
(iii) Gasphasenhydrierung des im vorhergehenden Schritt erhaltenen 1,2,3,3,3-Pentafluor-1-propens zu 1,1,1,2,3-Pentafluorpropan in Gegenwart einer überstöchiometrischen Menge Wasserstoff und eines Katalysators in einem Reaktor;
(iv) Reinigung des im vorhergehenden Schritt erhaltenen 1,1,1,2,3-Pentafluorpropans zum Erhalt eines Stroms, der mehr als 98 Gew.-% 1,1,1,2,3-Pentafluorpropan umfasst;
(v) Dehydrofluorierung des gereinigten 1,1,1,2,3-Pentafluorpropans zu 2,3,3,3-Tetrafluor-1-propen in Gegenwart eines Dehydrofluorierungskatalysators oder mit Hilfe einer Mischung von Wasser und Kaliumhydroxid;
(vi) Reinigung des im vorhergehenden Schritt erhaltenen 2,3,3,3-Tetrafluor-1-propens.

2. Verfahren nach Anspruch 1, **dadurch gekennzeichnet, dass** die Reinigung des 1,1,1,2,3-Pentafluorpropans in (iv) einen Schritt der Destillation bei einem Druck zwischen 1 und 20 bar absolut umfasst.

3. Verfahren nach Anspruch 1 oder 2, **dadurch gekennzeichnet, dass** die in Schritt (iv) zu entfernenden Verbindungen eine Siedetemperatur aufweisen, die mindestens 20 °C oder sogar mindestens 10 °C unter der Siedetemperatur von 1,1,1,2,3-Pentafluorpropan liegt.

4. Verfahren nach dem vorhergehenden Anspruch, **dadurch gekennzeichnet, dass** die zu entfernenden Verbindungen aus 1,2,3,3,3-Pentafluorpropen, 1,1,3,3,3-Pentafluoropropen und 1,1,1,2-Tetrafluorethan ausgewählt werden.

5. Verfahren nach einem der vorhergehenden Ansprüche, **dadurch gekennzeichnet, dass** das Kaliumhydroxid zwischen 20 und 75 Gew.-% der Wasser-Kaliumhydroxid-Mischung von Schritt (ii) und/oder (v) ausmacht.

6. Verfahren nach einem der vorhergehenden Ansprüche, bei dem Schritt (ii) und/oder Schritt (v) bei einer Temperatur zwischen 80 und 180 °C, vorzugsweise zwischen 145 und 165 °C, durchgeführt wird bzw. werden.

7. Verfahren nach einem der vorhergehenden Ansprüche, **dadurch gekennzeichnet, dass** der gasförmige Austragsstrom aus Schritt (i) und/oder (iii) einem Schritt der teilweisen Kondensation unterworfen wird bzw. werden.

8. Verfahren nach einem der vorhergehenden Ansprüche, **dadurch gekennzeichnet, dass** der Katalysator aus Schritt (i) und/oder Schritt (iii) zwischen 0,05 und 10 Gew.-% und vorzugsweise zwischen 0,1 und 5 Gew.-% auf Aluminiumoxid oder Kohle geträgertes Palladium umfasst.

9. Verfahren nach einem der vorhergehenden Ansprüche, **dadurch gekennzeichnet, dass** der Schritt (iii) bei einer Temperatur am Eingang des Reaktors zwischen 80 und 140 °C durchgeführt wird.

10. Verfahren nach einem der vorhergehenden Ansprüche, **dadurch gekennzeichnet, dass** in Schritt (vi) ein Strom, der mehr als 99,8 Gew.-% 2,3,3,3-Tetrafluorpropen umfasst, gebildet wird.

## Claims

1. Process for the preparation of 2,3,3,3-tetra-fluoro-1-propene comprising the following stages:
(i) gas-phase hydrogenation of hexafluoropropylene to give 1,1,1,2,3,3-hexafluoropropane in the presence of a superstoichiometric amount of hydrogen and of a catalyst in a reactor;
(ii) dehydrofluorination of the 1,1,1,2,3,3-hexafluoropropane obtained in the preceding stage to give 1,2,3,3,3-pentafluoro-1-propene in the presence of a dehydrofluorination catalyst or using a water and potassium hydroxide mixture;
(iii) gas-phase hydrogenation of the 1,2,3,3,3-pentafluoro-1-propene obtained in the preceding stage to give 1,1,1,2,3-pentafluoropropane in the presence of a superstoichiometric amount of hydrogen and of a catalyst in a reactor;
(iv) purification of the 1,1,1,2,3-pentafluoropropane obtained in the preceding stage in order to obtain a stream comprising more than 98% by weight of 1,1,1,2,3-pentafluoropropane;
(v) dehydrofluorination of the purified 1,1,1,2,3-pentafluoropropane to give 2,3,3,3-tetra-fluoro-1-propene in the presence of a dehydrofluorination catalyst or using a water and potassium hydroxide mixture;
(vi) purification of the 2,3,3,3-tetrafluoro-1-propene obtained in the preceding stage.

2. Process according to Claim 1, **characterized in that** the purification of the 1,1,1,2,3-pentafluoropropane in (iv) comprises a distillation stage at a pressure of between 1 and 20 bar absolute.

3. Process according to Claim 1 or 2, **characterized in that** the compounds to be removed in stage (iv) have a boiling point lower by at least 20°C, indeed even lower by at least 10°C, with respect to that of 1,1,1,2,3-pentafluoropropane.

4. Process according to the preceeding claim, **characterized in that** the compounds to be removed are chosen from 1,2,3,3,3-pentafluoropropene, 1,1,3,3,3-pentafluoropropene and 1,1,1,2-tetrafluoroethane.

5. Process according to any one of the preceding claims, **characterized in that** the potassium hydroxide represents between 20 and 75% by weight of the water/potassium hydroxide mixture of stage (ii) and/or (v) .

6. Process according to any one of the preceding claims, in which stage (ii) and/or stage (v) is or are carried out at a temperature of between 80 and 180°C, preferably of between 145 and 165°C.

7. Process according to any one of the preceding claims, **characterized in that** the gaseous output stream resulting from stage (i) and/or (iii) is or are subjected to a stage of partial condensation.

8. Process according to any one of the preceding claims, **characterized in that** the catalyst of stage (i) and/or stage (iii) comprises between 0.05 and 10% by weight and preferably between 0.1 and 5% by weight of palladium supported on alumina or carbon.

9. Process according to any one of the preceding claims, **characterized in that** stage (iii) is carried out at a temperature at the inlet of the reactor of between 80 and 140°C.

10. Process according to any one of the preceding claims, **characterized in that** stage (vi) forms a stream comprising more than 99.8% by weight of 2,3,3,3-tetrafluoropropene.
